# EUROPÄISCHE PATENTSCHRIFT

(11) **EP 2 695 721 B1**
(45) Veröffentlichungstag und Bekanntmachung des Hinweises auf die Patenterteilung: **23.12.2015**
(21) Anmeldenummer: 13179774.8
(22) Anmeldetag: 08.08.2013
(51) Int. Cl.: B29C 49/46, A61L 2/00, F16J 15/44

(54) **Abdichteinrichtung mit Kompensationsmöglichkeit für mehrere Bewegungsrichtungen**
Sealing device with compensation facility for multiple directions of movement
Dispositif d'étanchéité avec possibilité de compensation pour plusieurs directions de mouvement

(30) Priorität: 10.08.2012 DE 102012107361
(43) Veröffentlichungstag der Anmeldung: 12.02.2014
(73) Patentinhaber: Krones AG, 93073 Neutraubling (DE)
(72) Erfinder: Geltinger, Florian, 93073 Neutraubling (DE); Neubauer, Michael, 93073 Neutraubling (DE)
(74) Vertreter: Bittner, Bernhard

(56) Entgegenhaltungen:
- EP-A1- 2 388 129
- EP-A2- 2 388 127
- EP-A2- 2 444 232
- DE-A1-102011 081 414

## Beschreibung

Die vorliegende Erfindung betrifft den Bereich der Getränke herstellenden Industrie und allgemein die Verpackungsindustrie. Aus dem Stand der Technik sind diverse Vorrichtungen und Verfahren bekannt, welche Behältnisse behandeln, wie beispielsweise Fülleinrichtungen, Verschließeinrichtungen, Transporteinrichtungen, Sterilisationseinrichtungen und dergleichen.

Bei der Abfüllung einiger Getränke ist man bestrebt, diese in einen Sterilraum vorzunehmen. In diesen Fällen werden die Behältnisse durch einen Sterilraum hindurch transportiert und während dieses Transports behandelt, beispielsweise befüllt, verschlossen oder sterilisiert. Dabei ist man bestrebt, eine möglichst zuverlässige Abdichtung dieses Sterilraumes gegenüber dessen Umgebung zu gewährleisten.

Viele der genannten Behandlungseinrichtungen weisen Behandlungselemente auf, welche im Inneren des Sterilraums Bewegungen durchführen, wie beispielsweise Verschließköpfe, welche die Verschlüsse auf die Behältnisse aufschrauben. Weiterhin ist man bestrebt, Antriebseinrichtungen, wie beispielsweise Motoren, aber auch Führungskurven, nach Möglichkeit außerhalb des Sterilraumes anzuordnen, um auf diese Weise das Verschmutzungsrisiko zu minimieren.

Um gleichwohl eine Bewegungsmöglichkeit für das im Inneren des Sterilraums angeordnete Behandlungselement zu schaffen, werden im Stand der Technik teilweise sogenannte Faltenbälge eingesetzt. Diese Faltenbälge sind dabei sowohl an einem Abschnitt des beweglichen Elements als auch beispielsweise an einem Abschnitt einer Gehäusewandung angeordnet und dichten damit den Bereich zwischen dem Behandlungselement und der besagten Wandung ab, sodass üblicherweise der Faltenbalg selbst auch eine Reinraumgrenze ausbildet. Ein Nachteil derartiger Faltenbälge besteht in der oftmals nur sehr eingeschränkten Bewegungsmöglichkeit So sind beispielsweise Faltenbälge bekannt, welche eine Bewegung in einer Längsrichtung des Behandlungselements erlauben und auch solche, die eine Bewegung quer zu dieser Richtung in einer Vorzugsrichtung ermöglichen. Bei einigen Behandlungselementen wäre jedoch darüber hinaus auch eine Drehbewegung des Behandlungselements wünschenswert. Herkömmliche Faltenbälge sind üblicherweise nicht geeignet, um derartige Drehbewegungen zu kompensieren.

Aus der EP 2 388129 A1 ist eine Vorrichtung zum Umformen von Kunststoffvorformlingen mit einem Sterilraum bekannt. Dabei wird ein Faltenbalg eingesetzt, um eine Blasformträgerbewegung abzudichten.

Der vorliegenden Erfindung liegt daher die Aufgabe zu Grunde, eine Abdichteinrichtung, insbesondere für Getränke- oder Behältnisse verarbeitende Anlagen herzustellen, welche auch eine Kompensation von Drehbewegungen erlaubt. Diese Aufgabe wird erfindungsgemäß durch eine Abdichteinrichtung sowie auch eine Vorrichtung zum Behandeln von Behältnissen nach den unabhängigen Ansprüchen erreicht. Vorteilhafte Ausführungsformen und Weiter bildungen sind Gegenstand der Unteransprüche.

Die Erfindung wird nachfolgend unter Bezugnahme auf eine Vorrichtung zum Umformen von Kunststoffvorformlingen beschrieben, es wird jedoch darauf hingewiesen, dass die vorliegende Erfindung auch bei anderen Vorrichtungen einsetzbar ist. Die Anmelderin behält sich vor, auch unter Bezugnahme auf andere Vorrichtungen insbesondere für die Getränke her stellende Industrie Schutz zu beanspruchen.

Eine erfindungsgemäße Vorrichtung zum Umformen von Kunststoffvorformlingen weist eine Abdichteinrichtung zum Abdichten von zwischen zueinander beweglich angeordneten Körpem (d.h. insbesondere zueinander relativbeweglichen Körpern) gebildeten Zwischenräumen auf. Diese weist einen aus einem elastischem und gasdichtem Material gebildeten und vollständig in einer Umfangsrichtung umlaufenden Grundkörper auf, durch den ein Abschnitt des ersten der beiden beweglichen Körper hindurch führbar ist. Daneben weist die Abdichteinrichtung einen ersten Endabschnitt auf, der an dem zweiten beweglichen Körper befestigbar ist sowie einen zweiten Endabschnitt, der an dem besagten ersten beweglichen Körper anlegbar ist. Weiterhin ist der zweite Endabschnitt gegenüber dem ersten Endabschnitt in einer ersten Querrichtung bewegbar, die zu der Längsrichtung in einem von 0°verschiedenen Winkel verläuft, bevorzugt zu der Längsrichtung in einem Winkel von wenigstens 60° verläuft und bevorzugt im Wesentlichen senkrecht zu der Längsrichtung steht. Bevorzugt ist der zweite Endabschnitt gegenüber dem ersten Endabschnitt in einer zweiten Querrichtung bewegbar, die zu der Längsrichtung und zu der ersten Querrichtung in einem von 0° verschiedenen Winkel verläuft und bevorzugt senkrecht ist.

Erfindungsgemäß ist an dem zweiten Endabschnitt der Abdichteinrichtung eine Gleitdichteinrichtung angeordnet, welche an dem ersten Körper derart anlegbar ist, dass eine Relativdrehung zwischen dem ersten Körper und dem zweiten Körper um einen vorgegebenen Drehwinkel ermöglicht ist.

Es wird daher erfindungsgemäß eine Kombination aus einem Faltenbalg mit einer Gleitabdichtung vorgeschlagen. Diese Abdichteinrichtung bietet den Vorteil, dass der erste bewegliche Körper insbesondere in beliebigen Raumrichtungen bewegbar ist und zusätzlich auch gegenüber dem zweiten Körper um einen vorgegebenen Winkel drehbar ist. Diese Drehbarkeit wird dabei insbesondere durch die oben erwähnte Gleitabdichtung gewährleistet. Auf diese Weise ist eine Abdichtung unter Beibehaltung der vollen Bewegbarkeit des bewegbaren Körpers ermöglicht. Auf diese Weise ist die Abdichteinrichtung für unterschiedlichste Bewegungselemente, wie sie im Bereich der Getränke- und/oder Verpackungsindustrie auftreten, geeignet. Damit kann die Abdichteinrichtung beispielsweise als Abdichteinrichtung für einen Verschließkopf zum Verschließen von Behältnissen eingesetzt werden, aber auch für Elemente, wie beispielsweise drehbare Sprühdüsen und dergleichen. Diese Abdichteinrichtung bzw. Bestandteile davon können aus einem flexiblen Material ausgestaltet sein, dies ist jedoch nicht unbedingt erforderlich. Bevorzugt ist wenigstens ein Bestandteil der Abdichteinrichtung aus einem Polymer hergestellt und insbesondere aus PTFE. So können insbesondere ringförmige Abdichtelemente aus PTFE hergestellt sein.

Vorzugsweise weist die Vorrichtung zum Umformen von Kunststoffvorformlingen zu Kunststoffbehältnissen eine Beaufschlagungseinrichtung auf, welche an eine Mündung der Kunststoffvorformlinge anlegbar ist, um diese mit einem gasförmigen Medium zu beaufschlagen. Weiterhin weist die Vorrichtung vorteilhaft eine Transporteinrichtung auf, um die Kunststoffvorformlinge auf einem vorgegebenen Transportpfad zu transportieren. Vorteilhaft weist die Transporteinrichtung einen bewegbaren und insbesondere drehbaren Träger auf, an dem eine Vielzahl von Umformungsstationen zum Umformen von Kunststoffvorformlingen zu Kunststoffbehältnissen angeordnet ist. Bei einer weiteren vorteilhaften Ausführungsform weisen diese Umformungsstationen jeweils einen stangenartigen Körper auf, der in einen Innenraum der Kunststoffvorformlinge einführbar ist, um diesen in seiner Längsrichtung zu dehnen.

Bevorzugt ist eine (abgedichtete) Drehung zwischen dem ersten Körper und dem zweiten Körper um einen Drehwinkel ermöglicht, der wenigstens 10°, bevorzugt wenigstens 20°, bevorzugt wenigstens 30 und bevorzugt wenigstens 40° beträgt. Bei einer bevorzugten Ausführungsform handelt es sich bei der Bewegung in der ersten Querrichtung um eine Schwenkbewegung eines der beiden beweglichen Körper und bevorzugt des ersten beweglichen Körpers um eine vorgegebene Schwenkachse. Dies bedeutet, dass bei dieser Bewegung bevorzugt ein Abstand zwischen dem ersten Endabschnitt und dem zweiten Endabschnitt der Abdichteinrichtung im Wesentlichen konstant bleibt. Bevorzugt ist der erste Körper gegenüber wenigstens einem Abschnitt der Abdichteinrichtung im Wesentlichen frei drehbar. Bevorzugt weist auch die Gleitdichteinrichtung einen drehfesten Teil und einen diesem drehfesten Teil gegenüber drehbaren Teil auf. Bevorzugt setzt sich die Abdichteinrichtung aus einem Faltenbalg und einer daran angeordneten Gleitdichteinrichtung zusammen.

Daneben kann die Abdichteinrichtung jedoch auch eine Bewegung in der Längsrichtung des ersten Körpers zwischen dem ersten Körper und dem zweiten Körper zulassen, insbesondere also eine Bewegung, bei der sich ein Abstand zwischen den Endabschnitten der Abdichteinrichtung verändert.

Bei einer vorteilhaften Ausführungsform weist der Grundkörper in seiner Längsrichtung unterschiedlich ausgebildete Abschnitte auf. Dabei ist es insbesondere möglich, dass ein erster Abschnitt des Grundkörpers in Längsrichtung zum Kompensieren von Längsbewegungen eingesetzt wird und ein anderer Abschnitt zum Kompensieren von Quer- bzw. Schwenkbewegungen. Vorteilhaft sind jedoch die einzelnen Abschnitte des Grundkörpers in einer Umfangsrichtung des Grundkörpers gleichbleibend ausgebildet. Dies erlaubt eine vereinfachte Fertigung der Abdichteinrichtung.

Bei einer weiteren vorteilhaften Ausführungsform weist ein Abschnitt des Grundkörpers eine umlaufende und bevorzugt mäandrierende (bzw. zick-zack-förmig- verlaufende) Wandung auf. Vorteilhaft ist dabei mäandrierende Wandung mit unterschiedlichen Wandstärken ausgebildet. Dabei können sich diese unterschiedlichen Wandstärken vorteilhaft (insbesondere periodisch) abwechseln. So ist es beispielsweise möglich, dass diejenigen Abschnitte der Wandung, die sich in der Längsrichtung des Grundkörpers erstrecken, eine größere Wanddicke haben als diejenigen Wandabschnitte, die sich in einer hierzu senkrecht verlaufenden Richtung bzw. in einer radialen Richtung des Grundkörpers erstrecken. Vorteilhaft sind die besagten Wandungsabschnitte mit den größeren und den geringeren Dicken jeweils senkrecht zueinander ausgebildet. Dabei ist es möglich, dass diejenigen Wandungsabschnitte, die sich in radialer Richtung erstrecken, insbesondere zum Aufnehmen von Bewegungen des bewegten Körpers in der oben besagten Längsrichtung dienen.

Bei einer weiteren vorteilhaften Ausführungsform weist ein Abschnitt des Grundkörpers eine umlaufende zylindrische Wand auf, an der insbesondere ringförmige Vorsprünge ausgebildet sind.

Bei einer weiteren vorteilhaften Ausführungsform ist die Abdichteinrichtung aus einem flexiblen Material hergestellt. Vorteilhaft ist die Abdichteinrichtung aus einem Material hergestellt, welches aus einer Gruppe von Materialien ausgewählt ist, welche Thermoplaste, Elastomere oder Metalle oder Kombinationen hieraus enthält. Diese Materialien sind sowohl für die Gleitabdichtung bzw. den Wellendichtring denkbar also auch für weitere Bestandteile der Abdichteinrichtung wie etwa einen Faltenbalg. Bei einer weiteren bevorzugten Ausführungsform ist die Abdichteinrichtung mehrteilig aufgebaut. So kann beispielsweise ein Wellendichtring, der einen Bestandteil der Abdichteinrichtung bildet, aus einem verschleißfesteren Werkstoff hergestellt sein, wobei unter anderem faserverstärkte Thermoplaste oder PTFE in Frage kommen, die bevorzugt einen Glasfaseranteil zwischen 5% und 50%, bevorzugt zwischen 10% und 30% aufweisen.

Bei einer weiteren vorteilhaften Ausführungsform weist die Gleitabdichteinrichtung einen Wellendichtring auf. Bevorzugt wirkt dieser Wellendichtring mit einer Dichtlippe zusammen, um so den Abdichteffekt auch bei Drehbewegungen zu erreichen. Dies bedeutet, dass in diesem Falle der bewegliche Körper gegenüber der Abdichteinrichtung drehbeweglich ist. Auf diese Weise können jedoch beliebige Drehbewegungen, das heißt auch Volldrehungen oder darüber hinaus gehende Drehungen, bewerkstelligt werden.

Bei einer weiteren vorteilhaften Ausführungsform ist die Abdichteinrichtung als Faltenbalg ausgeführt, während bevorzugt an einem Ende bzw. an einem endseitigen Abschnitt ein Aufnahmeflansch vorgesehen ist. Auf dieser Seite wird die Abdichteinrichtung an einem abzudichtenden Bauteil, wie beispielsweise einer Reinraumeinhausung befestigt. An dem anderen Endabschnitt ist vorteilhaft der oben genannte Wellendichtring angeordnet. Das gesamte Bauteil bzw. die Abdichteinrichtung kann dabei einteilig aufgebaut sein oder aus mehreren Teilen bestehen.

Vorteilhaft ist damit der Grundkörper der Abdichteinrichtung wenigstens abschnittsweise als Faltenbalg ausgebildet. Diese Ausführung als Faltenbalg dient dabei insbesondere zum Kompensieren von Längsbewegungen.

Bei einer weiteren vorteilhaften Ausführungsform ist die Abdichteinrichtung einteilig ausgebildet. So ist es möglich, dass die Abdichteinrichtung aus einem einzigen Werkstoff besteht.

Es wäre jedoch auch möglich, dass der Grundkörper und die Gleitabdichtung separate jedoch miteinander verbundene Bauteile sind. So wäre es möglich, dass zwei oder mehrere Werkstoffe miteinander verbunden sind. Vorteilhaft sind die Werkstoffe miteinander stoffschlüssig verbunden, also beispielsweise durch Sintern.

Weiterhin wäre es jedoch möglich, dass bei einer mehrteiligen Ausführung der Grundkörper und der Wellendichtring separate Bauteile sind, die miteinander verbunden sind, beispielsweise miteinander verschraubt sind. Diese Konstruktion bietet Vorteile, dass auch überlagerte Bewegungen, das heißt Rotationen und Schwenkbewegungen und zusätzlich eine Translation zuverlässig abgedichtet werden können.

Bei einer weiteren vorteilhaften Ausführungsform ist an dem ersten beweglichen Körper ein umlaufender Vorsprung angeordnet, an dem wenigstens ein Abschnitt der Gleitabdichtung angeordnet ist. Allgemein ist vorteilhaft die Abdichteinrichtung bzw. der zweite Endabschnitt im Wesentlichen nicht längs verschiebbar gegenüber dem beweglichen Bauteil. Mit anderen Worten ist bevorzugt zwar eine Drehbewegung des beweglichen Elements gegenüber der Abdichteinrichtung möglich, nicht jedoch eine Längsverschiebung des beweglichen Elements gegenüber der Abdichteinrichtung. So kann es sein, dass der besagte umlaufende Vorsprung von dem zweiten Endabschnitt der Abdichteinrichtung aufgenommen wird. Es wäre jedoch auch möglich, dass an dem beweglichen Körper eine umlaufende Nut ausgebildet ist, in welche ein Abschnitt der Abdichteinrichtung eingreift.

Bei einer weiteren vorteilhaften Ausführungsform weist die Abdichteinrichtung eine wenigstens teilweise umlaufende und sich zumindest auch in einer radialen Richtung (also ggfs. auch schräg bezüglich der radialen Richtung) erstreckende Nut zur Aufnahme eines Abschnitts des ersten Körpers auf. Bevorzugt ist diese Nut in einer Umfangsrichtung der Abdichteinrichtung umlaufend ausgebildet und weist bevorzugt einen kreisförmigen Querschnitt auf.

Bei einer weiteren vorteilhaften Ausführungsform steht der erste Endabschnitt in gasdichter Verbindung mit dem zweiten Körper und/oder der zweite Endabschnitt in gasdichter Verbindung mit dem ersten beweglichen Körper. Dabei können bevorzugt auch die Schnittstellen bei einer mehrteiligen Ausführung der Abdichteinrichtung gasdicht ausgeführt sein.

Die vorliegende Erfindung ist weiterhin auf eine Vorrichtung zum Behandeln von Behältnissen gerichtet, welche eine Transporteinrichtung aufweist, welche die zu behandelnden Behältnisse entlang eines vorgegebenen Transportpfads transportiert. Weiterhin weist die Vorrichtung einen Reinraum auf, innerhalb dessen die Behältnisse transportiert werden, wobei dieser Reinraum mittels wenigstens einer Wandung gegenüber einer Umgebung abgetrennt ist. Weiterhin ist ein gegenüber der Wandung bewegliches Behandlungselement vorgesehen, welches die Behältnisse in einer vorgegebenen Weise behandelt, wobei ein Antrieb zum Bewegen dieses Behandlungselements wenigstens abschnittsweise außerhalb des Reinraums angeordnet ist und das Behandlungselement wenigstens abschnittsweise in den Reinraum hinein ragt.

Weiterhin ist eine Abdichteinrichtung zum Abdichten eines zwischen dem Behandlungselement und der Wandung des Reinraums auftretenden Zwischenraums vorgesehen, welche einen aus einem elastischen und gasdichten Material gebildeten und vollständig in einer Umfangsrichtung um wenigstens einen Abschnitt des Behandlungselements umlaufenden Grundkörper aufweist, durch den dieser besagte Abschnitt des Behandlungselements hindurch geführt ist.

Weiterhin weist die Abdichteinrichtung einen ersten Endabschnitt, der an der Wandung (vorteilhaft gas- und flüssigkeitsdicht) befestigt ist sowie einen zweiten Endabschnitt, der an dem Behandlungselement angelegt ist, auf. Dabei ist der zweite Endabschnitt gegenüber dem ersten Endabschnitt bewegbar.

Erfindungsgemäß ist an dem zweiten Endabschnitt der Abdichteinrichtung ein Dichtungselement angeordnet, welches derart an das Behandlungselement anlegbar ist, dass eine Relativdrehung zwischen dem Behandlungselement und der Wandung um einen vorgegebenen Drehwinkel ermöglicht ist. Vorteilhaft ist daher als Dichtungselement eine Gleitabdichteinrichtung vorgesehen.

Im Rahmen dieser Erfindung wird daher vorgeschlagen, ein Abdichtelement der oben beschriebenen Art insbesondere für Vorrichtungen zum Behandeln von Behältnissen einzusetzen. Der oben erwähnte erste Körper ist damit hier das Behandlungselement und der zweite Körper die Wandung. Damit bildet bevorzugt die Abdichteinrichtung auch eine Grenze des Reinraums aus.

Vorteilhaft ist der zweite Endabschnitt gegenüber dem ersten Endabschnitt in einer ersten Querrichtung bewegbar, die zur Längsrichtung senkrecht ist und vorteilhaft ist der zweite Endabschnitt auch gegenüber dem ersten Endabschnitt in einer zweiten Querrichtung bewegbar, die zu der Längsrichtung und zu der ersten Querrichtung senkrecht ist.

Damit wird auch, wie oben bereits beschrieben, vorgeschlagen, dass ein Endabschnitt gegenüber dem anderen im Wesentlichen in beliebigen Raumrichtungen bewegbar ist und zusätzlich auch noch der besagte Abschnitt des Behandlungselements drehbar gegenüber dem Abdichtelement ist.

Vorteilhaft ist die Vorrichtung zum Behandeln von Behältnissen aus einer Gruppe von Vorrichtungen ausgewählt, welche Fülleinrichtungen zum Befüllen von Behältnissen, Sterilisationseinrichtungen zum Sterilisieren von Behältnissen, Verschließeinrichtungen zum Verschließen von Behältnissen, Etikettiereinrichtungen und dergleichen enthält. Weiterhin kann es sich bei der Vorrichtung zum Behandeln von Behältnissen auch um eine Umformungseinrichtung zum Umformen von Kunststoffvorformlingen zu Kunststoffbehältnissen oder auch um eine Erwärmungseinrichtung zum Erwärmen von Kunststoffbehältnissen handeln.

Bei dem Antrieb handelt es sich vorteilhaft um einen elektromotorischen Antrieb. Es wäre jedoch auch möglich, dass hier zumindest für einige der Bewegungen, wie etwa eine Hubbewegung, als Bewegungseinrichtung eine (insbesondere stationär angeordnete) Führungskurve vorgesehen ist.

Vorteilhaft weist der Reinraum eine stehende Wandung auf und eine gegenüber dieser stehenden Wandung bewegliche Wandung. Vorteilhaft ist die bewegliche Wandung um eine vorgegebene Drehachse drehbar. Daneben kann das Behandlungselement auch an einem drehbaren Träger angeordnet sein.

Vorteilhaft sind die besagten stehenden und beweglichen Wandungen des Reinraums bezüglich einander abgedichtet, beispielsweise unter Verwendung wenigstens einer umlaufenden Dichtungseinrichtung, welche einen mit einer Flüssigkeit gefüllten Kanal aufweist. Derartige Dichtungseinrichtungen sind aus dem Stand der Technik als sogenannte Wasserschlösser bekannt.

Bei einer weiteren vorteilhaften Ausführungsform ist das Behandlungselement aus einer Gruppe von Behandlungselementen ausgewählt, welche Verschließelemente zum Verschließen von Behältnissen mit Verschlüssen, Füllelemente zum Befüllen von Behältnissen, Sterilisationselemente zum Sterilisieren von Behältnissen, Halteelemente zum Halten von Behältnissen und dergleichen enthält.

Weitere Vorteile und Ausführungsformen ergeben sich aus den beigefügten Zeichnungen.

Darin zeigen:
- Fig. 1: eine schematische Darstellung einer Abdichteinrichtung;
- Fig. 2: eine Darstellung der Abdichteinrichtung unter Berücksichtigung der möglichen Bewegungen;
- Fig. 3: eine Darstellung einer erfindungsgemäßen Abdichteinrichtung in einem vorgegebenen Bewegungszustand;
- Fig. 4: eine Schnittdarstellung der in Fig. 3 gezeigten Abdichteinrichtung; und
- Fig. 5: eine Darstellung einer Behandlungseinrichtung mit einer erfindungsgemäßen Abdichteinrichtung.

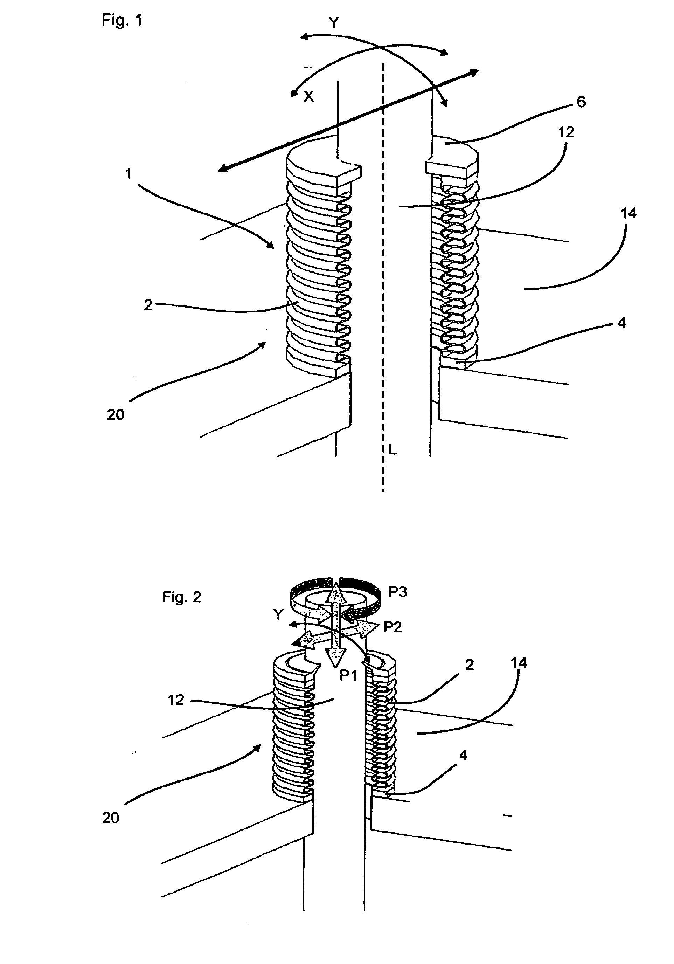

Fig. 1 zeigt eine Abdichteinrichtung 1 in einer ersten stark schematischen Darstellung. Dabei ist eine Wandung 14 bzw. ein Wandungselement 14 vorgesehen sowie ein gegenüber dieser Wandung 14 bewegliches Bewegungselement 12, wobei der Zwischenraum zwischen der Wandung und dem Bewegungselement 12 durch die Abdichteinrichtung 1 abgedichtet werden soll. So ist es beispielsweise möglich, dass sich unterhalb der Wandung 14 ein Reinraum befindet und oberhalb der Wandung eine (unsterile) Umgebung. Vorteilhaft weist die Wandung 14 eine Öffnung 15 auf (siehe Fig. 2), die einen größeren Querschnitt aufweist als das Bewegungselement und durch welche das Bewegungselement hindurchgeführt ist.

Die Abdichteinrichtung 1 weist einen ersten Endabschnitt 4 auf, der bevorzugt fest an der Wandung 14 angeordnet ist. Ein zweiter Endabschnitt 6 der Abdichteinrichtung liegt an dem Bewegungselement 12, welches hier einen stangenartigen Abschnitt aufweist, an. Das Bezugszeichen X kennzeichnet eine mögliche Bewegungsrichtung des Bewegungselements 12. Dabei ist es möglich, dass das Bewegungselement 12 in seiner Gesamtheit bewegt wird, es wäre jedoch auch möglich, dass das Bewegungselement 12 in der Richtung X geschwenkt wird. Das Bezugszeichen 2 kennzeichnet einen Grundkörper der Abdichtabrichtung 1. Damit bewegt sich bei dieser Schwenkbewegung bevorzugt der zweite Endabschnitt der Abdichteinrichtung 20 gegenüber dem ersten Endabschnitt der Abdichteinrichtung entlang einer Kreisbahn. Daneben wäre es denkbar, dass das Bewegungselement auch in einer zweiten Richtung Y, die hier senkrecht zu der ersten Richtung steht, beweglich ist. Dabei könnte es sich ebenfalls um eine Schwenkbewegung handeln. Bevorzugt ist bei dieser Ausführungsform der zweite Endabschnitt 6 gegenüber dem ersten Endabschnitt 4 in einer kugelförmigen Ebene bzw. Fläche bewegbar.

Fig. 2 zeigt eine Darstellung ähnlich der in Fig. 1 gezeigten Darstellung, wobei hier zusätzlich die möglichen Bewegungsrichtungen des Bewegungselements 12 dargestellt sind. Man erkennt, dass das Bewegungselement 12 sich sowohl in der Richtung P1 bewegen kann, das heißt in der in Fig. 1 gezeigten Längsrichtung L des Bewegungselements 12 als auch in der Richtung P2 und auch in einer (hier nicht gezeigten) Richtung P4, die auch senkrecht zu Richtung P1 und P2 steht. Auch ist hier wieder eine Bewegbarkeit in den bereits in Fig. 1 gezeigten Richtungen X und Y, die geradlinig bzw. auf einer Kreisbahn verlaufen, möglich.

Das Bezugszeichen P3 kennzeichnet eine mögliche Drehbewegung des Bewegungselements 12. An dem Bewegungselement könnte beispielsweise ein Verschließkopf zum Verschließen von Behältnissen angeordnet sein. Weiterhin könnte außerhalb eines Reinraums, das heißt hier oberhalb der Wandung 14, eine Antriebseinrichtung vorgesehen sein, welche das Bewegungselement 12 bewegt.

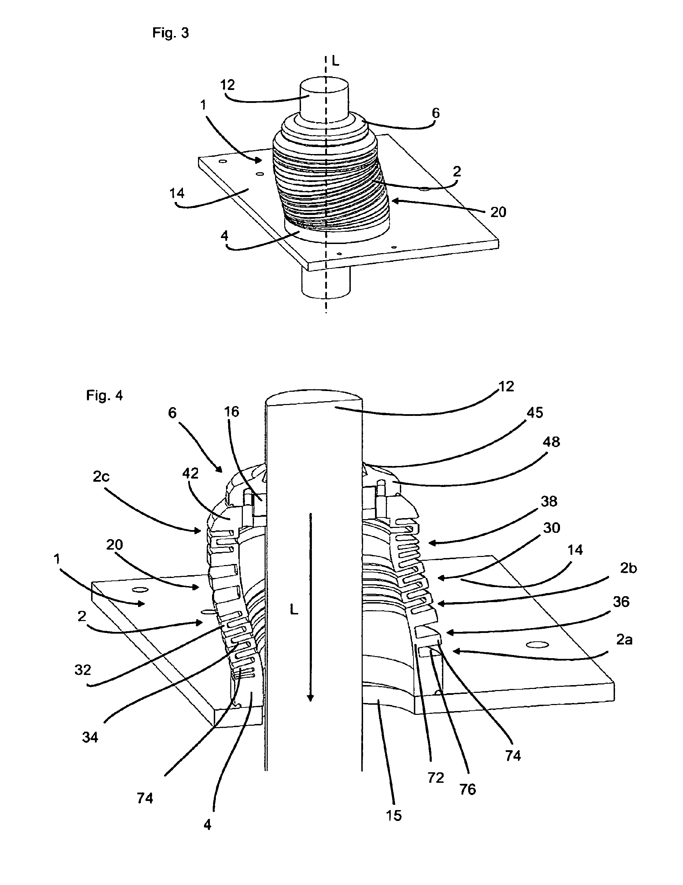

Fig. 3 zeigt eine perspektivische Darstellung einer erfindungsgemäßen Abdichteinrichtung, die an einer Wandung 14 angeordnet ist. Man erkennt, dass der Grundkörper 2 hier als Faltenbalg ausgebildet ist und der erste Endabschnitt 4 an der Wandung 14 angeordnet ist. Dabei kann der Endabschnitt 4 als Anschlussflansch ausgebildet sein, der beispielsweise an die Wandung 14 angeklebt ist. Das Bezugszeichen 20 kennzeichnet eine Gleitabdichteinrichtung, die an das Bewegungselement 12 bzw. eine Außenoberfläche des Bewegungselements 12 anlegbar ist.

Der zweite Endabschnitt 6 ist hier bevorzugt als Wellendichtring ausgebildet, der eine Relativdrehung des Bewegungselements 12 gegenüber dem Grundkörper 2 ermöglicht. Man erkennt, dass der Grundkörper 2 hier ziehharmonikaartig verbogen werden kann, um so die Bewegungen des Bewegungselements 12 insbesondere in der Ebene der Wandung 14 zu ermöglichen.

Fig. 4 zeigt eine Schnittdarstellung der in Fig. 3 gezeigten Vorrichtung. Man erkennt hier wiederum den ersten Endabschnitt 4, der an der Wandung 14 angeordnet ist. Der zweite Endabschnitt 6 der Abdichteinrichtung 1 liegt hier, wie oben erwähnt, teilweise an dem Bewegungselement 12 an. Zu diesem Zweck kann an dem Bewegungselement 12 ein umlaufender Vorsprung bzw. Kragen 16 ausgebildet sein. Dieser umlaufende Kragen 16 greift in eine in dem zweiten Endabschnitt 6 gebildete Ausnehmung ein. Zu diesem Zweck kann der zweite Endabschnitt 6 ein erstes Aufnahmeelement 42 aufweisen, sowie ein beispielsweise an dieses erste Aufnahmeelement 42 anschraubbares zweites Aufnahmeelement 48, wobei der umlaufende Vorsprung 16 zwischen diesen beiden Aufnahmeelementen 42 und 48 einfügbar ist. Das Bezugszeichen 45 kennzeichnet eine Dichtlippe, die an dem Bewegungselement 12 anliegen kann. Auf diese Weise wird zwischen dem Bewegungselement 12 und dem zweiten Endabschnitt 6 eine Abdichtung, z.B. eine Gleitabdichtung (z.B. durch einen Wellendichtring) ermöglicht, welche auch eine Relativdrehung des Bewegungselements 12 zulässt.

Man erkennt weiterhin, dass der Grundkörper 2 hier unterschiedliche Gestaltungen aufweist. In einem unteren Bereich 2a weist der Grundkörper eine umlaufende Wandung 72 auf sowie ringförmige Vorsprünge 74, die sich hier radial nach außen erstrecken. Denkbar wären auch entsprechende Vorsprünge, die sich radial nach innen erstrecken.

Dieser Abschnitt 2a dient hier insbesondere zur Aufnahme von Transversalbewegungen, das heißt Bewegungen, welche sich in der Ebene der Wandung 14 erstrecken. Zwischen diesen ringförmigen Vorsprüngen 74 sind jeweils Nuten 76 ausgebildet.

Das Bezugszeichen 2b kennzeichnet einen weiteren Abschnitt des Grundkörpers, der hier als zickzack förmig ausgestaltete Wand ausgebildet ist. Diese Wand weist hier Wandungsabschnitte 32 auf, die sich in einem unbelasteten Zustand des Bewegungselements, das heißt in einem Zustand, in dem dieser in einer mittleren Stellung bezüglich der Wandung ist, senkrecht verlaufen, das heißt in der Längsrichtung L. Daneben sind zweite Wandungsabschnitte 34 vorgesehen, die sich hier in eine bezüglich der Längsrichtung L radialen Richtung erstrecken. Insbesondere diese Abschnitte 34 dienen dabei zur Aufnahme bzw. Kompensation von Bewegungen des Bewegungselements 12 in seiner Längsrichtung, das heißt insbesondere für Hubbewegungen.

Ein dritter Abschnitt 2c des Grundkörpers ist wiederum aufgebaut wie der erste Abschnitt 2 a und dient, wie in Fig. 4 gezeigt, wiederum insbesondere zur Aufnahme von Transversalbewegungen. Auf diese Weise wird eine S-förmige Verformung der Abdichteinrichtung 2 bei einer Transversalbewegung des Bewegungselements 12 ermöglicht.

Es wäre jedoch auch denkbar, dass sämtliche Wandungen des Faltenbalgs bzw. der Abdichteinrichtung gleichartig ausgebildet sind (bevorzugt sowohl in der Längsrichtung L als auch in der (um die Längsrichtung verlaufenden) Umfangsrichtung. Eine derartige Ausführungsform ist auch bevorzugt.

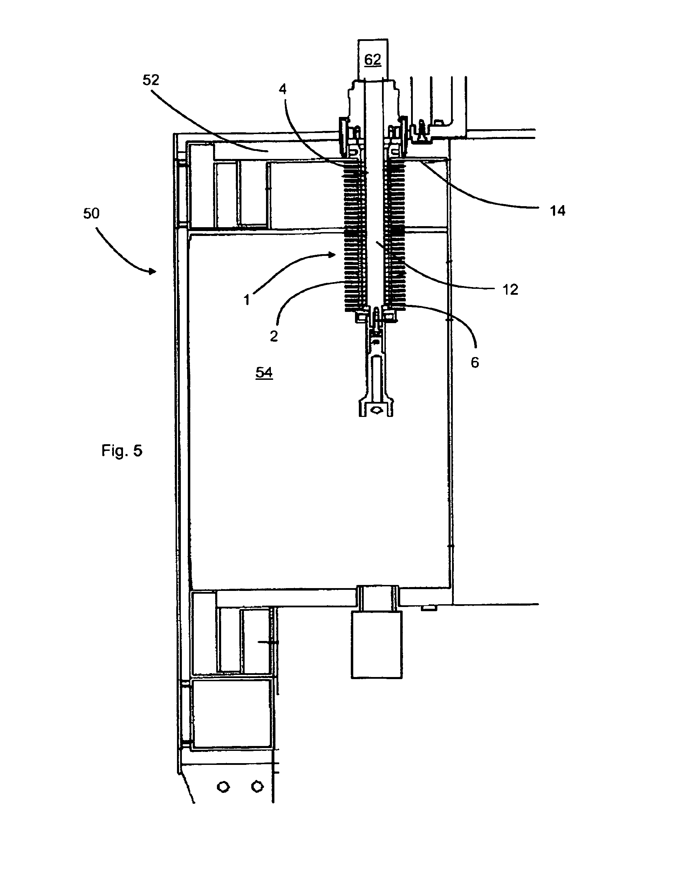

Fig. 5 zeigt eine schematische Darstellung einer Vorrichtung zum Behandeln von Behältnissen, bei der die in den oben genannten Figuren gezeigte Abdichteinrichtung eingesetzt ist. Bei der in Fig. 5 gezeigten grob schematischen Darstellung handelt es sich nicht um eine konkrete Vorrichtung 50, diese Darstellung dient lediglich der Veranschaulichung der Erfindung. Die Vorrichtung weist ein Behandlungselement 12 auf, welches stangenartig ausgebildet ist und sich durch eine Wandung 14 hindurch in einen Reinraum 54 erstreckt. An dem unteren Ende des Behandlungselements 12 kann ein Halteelement angeordnet sein, welches ein Behältnis (nicht gezeigt) hält, denkbar wäre jedoch auch ein Betätigungselement, welches geeignet ist, einen bestimmten Vorgang auszulösen, wie etwa einen Verriegelungs- oder einen Entriegelungsvorgang von Blasformen bzw. von Formträgern oder Blasstationen bei einer Streckblasmaschine. Die oben erwähnten Bewegungen in der X - bzw.Y - Richtung ist in Fig. 5 nicht dargestellt.

Bevorzugt handelt es sich bei der Vorrichtung 50 um eine Vorrichtung zum Umformen von Kunststoffvorformlingen zu Kunststoffbehältnissen, wie insbesondere eine Streckblasmaschine. Bei den zu behandelnden Behältnissen handelt es sich insbesondere um Kunststoffvorformlinge oder Kunststoffflaschen. Das Behandlungselement 12 entspricht dabei dem oben erwähnten ersten Körper und die Wandung 14 dem oben erwähnten zweiten Körper.

Das Bezugszeichen 52 kennzeichnet eine Transporteinrichtung, welche insbesondere zum Transportieren der zu behandelnden Behältnisse dient. Dabei kann es sich bei dieser Transporteinrichtung um ein Trägerrad, insbesondere ein Blasrad handeln, an dem eine Vielzahl von Behandlungseinrichtungen zum Behandeln von Kunststoffbehältnissen angeordnet ist. Bevorzugt weist dabei jede dieser Behandlungseinheiten ein Behandlungselement 12 auf.

Das Bezugszeichen 1 kennzeichnet eine Abdichteinrichtung. Ein erster Endabschnitt 4 ist dabei an der Wandung 14 angeordnet und an einem zweiten Endabschnitt 6 ist das Behandlungselement 12 derart anlegbar, dass es um seine Längsachse drehbar ist. Das Bezugszeichen 62 kennzeichnet einen Antrieb bzw. eine Antriebseinrichtung zum Antreiben des Behandlungselements 12, welche außerhalb des Reinraums 54 angeordnet ist. Nicht dargestellt in dieser Figur ist die durch die erfindungsgemäße Abdichteinrichtung ermöglichte Schwenkbarkeit des Behandlungselements 12. Dieses Behandlungselement könnte beispielsweise um eine Achse schwenkbar sein, welche senkrecht zu dem Behandlungselement in der Ebene der Wandung verläuft. Das Bezugszeichen 2 kennzeichnet wiederum den faltenbalgartigen Grundkörper der Abdichteinrichtung 1.

### Bezugszeichenliste

- 1: Abdichteinrichtung
- 2: Grundkörper
- 2a-2c: Abschnitte des Grundkörpers
- 4: erster Endabschnitt der Abdichteinrichtung
- 6: zweiter Endabschnitt der Abdichteinrichtung
- 12: Bewegungselement, erster Körper
- 14: Wandung, zweiter Körper
- 15: Öffnung
- 16: Vorsprung, Kragen
- 20: Gleitabdichteinrichtung
- 32: Wandungsabschnitte
- 34: Abschnitte
- 42: Aufnahmeelement
- 45: Dichtlippe
- 48: Aufnahmeelement
- 50: Vorrichtung
- 54: Reinraum
- 52: Transporteinrichtung
- 62: Antrieb
- 72: umlaufende Wandung
- 74: ringförmige Vorsprünge
- 76: Nuten
- X, Y: Bewegungsrichtungen
- L: Längsrichtung
- P1-P4: Bewegungsrichtungen

## Patentansprüche

1. Vorrichtung (50) zum Umformen von Kunststoffvorformlingen (10) zu Kunststoffbehältnissen (20) mit einer Abdichteinrichtung (1) zum Abdichten von zwischen zueinander beweglichen Körpern (12, 14) gebildeten Zwischenräumen, wobei die Abdichteinrichtung einen aus einem elastischen und gasdichten Material gebildeten und vollständig in einer Umfangsrichtung umlaufenden Grundkörper (2) aufweist durch den ein Abschnitt des ersten der beiden Körper (12, 14) hindurchführbar ist, sowie einen ersten Endabschnitt (4), der an dem zweiten Körper (14) befestigbar ist und einen zweiten Endabschnitt (6), der an den ersten beweglichen Körper (12) anlegbar ist, wobei der zweite Endabschnitt (6) gegenüber dem ersten Endabschnitt (4) in einer ersten Querrichtung (X) bewegbar ist, die zu der Längsrichtung (L) in einem von 0° verschiedenen Winkel verläuft
**dadurch gekennzeichnet, dass**
an dem zweiten Endabschnitt (6) der Abdichteinrichtung eine Gleitabdichteinrichtung (20) angeordnet ist, welche an den ersten beweglichen Körper (12) anlegbar ist, derart, dass eine Relativdrehung zwischen dem ersten Körper (12) und dem zweiten Körper (14) um einen vorgegebenen Winkel ermöglicht ist.

2. Vorrichtung (50) nach Anspruch 1,
**dadurch gekennzeichnet, dass**
die Gleitabdichteinrichtung (20) einen Wellendichtring aufweist.

3. Vorrichtung (50) nach Anspruch 1,
**dadurch gekennzeichnet, dass**
die erste Querrichtung (X) im Wesentlichen senkrecht zu der Längsrichtung (L) ist.

4. Vorrichtung (50)nach wenigstens einem der vorangegangenen Ansprüche,
**dadurch gekennzeichnet, dass**
der Grundkörper (2) wenigstens abschnittsweise als Faltenbalg ausgebildet ist.

5. Vorrichtung (50)nach wenigstens einem der vorangegangenen Ansprüche,
**dadurch gekennzeichnet, dass**
an dem ersten Endabschnitt (4) ein Aufnahmeflansch (42) angeordnet ist.

6. Vorrichtung (50)nach wenigstens einem der vorangegangenen Ansprüche,
**dadurch gekennzeichnet, dass**
die Abdichteinrichtung (1) einteilig ausgebildet ist.

7. Vorrichtung (50)nach wenigstens einem der vorangegangenen Ansprüche,
**dadurch gekennzeichnet, dass**
der Grundkörper (2) und die Gleitabdichtung (20) separat miteinander verbundene Elemente sind.

8. Vorrichtung (50)nach wenigstens einem der vorangegangenen Ansprüche,
**dadurch gekennzeichnet, dass**
an dem ersten beweglichen Körper (12) ein umlaufender Vorsprung (16) angeordnet ist, an dem wenigstens ein Abschnitt der Gleitabdichtung (20) angeordnet ist.

9. Vorrichtung (50)nach wenigstens einem der vorangegangenen Ansprüche,
**dadurch gekennzeichnet, dass**
der zweite Endabschnitt (6) gegenüber dem ersten Endabschnitt (4) in einer zweiten Querrichtung (Y) bewegbar ist, die zu der Längsrichtung (L) und zu der ersten Querrichtung (X) in einem von 0° verschiedenen Winkel verläuft.

10. Vorrichtung (50)nach wenigstens einem der vorangegangenen Ansprüche,
**dadurch gekennzeichnet, dass**
der erste Endabschnitt (4) in gasdichter Verbindung mit dem zweiten Körper (14) und/oder der zweite Endabschnitt (6) in gasdichter Verbindung mit dem ersten beweglichen Körper (12) steht.

11. Vorrichtung (50) zum Behandeln von Behältnissen, mit einer Transporteinrichtung (52) welche die zu behandelnden Behältnisse entlang eines vorgegebenen Transportpfades transportiert, mit einem Reinraum (54), innerhalb dessen die Behältnisse transportiert werden, wobei dieser Reinraum (54) mittels wenigstens einer Wandung (14) gegenüber einer Umgebung abgetrennt ist, mit einem gegenüber der Wandung (14) beweglichen Behandlungselement (12), welches die Behältnisse in einer vorgegebenen Weise behandelt, wobei ein Antrieb (62) zum Bewegen dieses Behandlungselements (12) wenigstens abschnittsweise außerhalb des Reinraums (54) angeordnet ist und das Behandlungselement (12) wenigstens abschnittsweise in den Reinraum hineinragt und mit einer Abdichteinrichtung (1) zum Abdichten eines zwischen dem Behandlungselement (12) und der Wandung (14) des Reinraums (54) auftretenden Zwischenraums, wobei die Abdichteinrichtung (1) einen aus einem elastischen und gasdichten Material gebildeten und vollständig in einer Umfangsrichtung umlaufenden Grundkörper (2) aufweist durch den ein Abschnitt des Behandlungselements (12) hindurchgeführt ist, sowie einen ersten Endabschnitt (4), der an der Wandung (14) befestigt ist und einen zweiten Endabschnitt (6), der an das Behandlungselement (12) anlegbar ist, **dadurch gekennzeichnet, dass** an dem zweiten Endabschnitt (6) der Abdichteinrichtung ein Dichtungselement (20) angeordnet ist, welches an dem Behandlungselement (12) anlegbar ist, derart, dass eine Relativdrehung zwischen dem ersten Körper (12) und dem zweiten Körper (14) um einen vorgegebenen Drehwinkel ermöglicht ist.

12. Vorrichtung (1) nach Anspruch 7,
**dadurch gekennzeichnet, dass**
der zweite Endabschnitt (6) gegenüber dem ersten Endabschnitt in einer ersten Querrichtung (X) bewegbar ist, die zu der Längsrichtung (L) im Wesentlichen senkrecht ist.

13. Vorrichtung (1) nach Anspruch 8,
**dadurch gekennzeichnet, dass**
das Behandlungselement aus einer Gruppe von Behandlungselementen ausgewählt ist, welche Verschließelemente zum Verschließen von Behältnissen mit Verschlüssen, Füllelemente zum Befüllen von Behältnissen , Sterilisationselemente zum Sterilisieren von Behältnissen, Halteelemente zum Halten von Behältnissen und dergleichen enthält.

## Claims

1. Apparatus (50) for shaping plastics material preforms (10) into plastics material containers (20) with a sealing device (1) for sealing off intermediate spaces formed between bodies (12, 14) arranged so as to be movable with respect to each other, wherein the sealing device has a main body (2) which is formed from a resilient and gas-tight material and which extends completely in a peripheral direction, through which a portion of the first of the two bodies (12, 14) is capable of being guided, and a first end portion (4), which is capable of being fastened to the second body (14), and a second end portion (6), which is capable of being set against the first movable body (12), wherein the second end portion (6) is movable with respect to the first end portion (4) in a first transverse direction (X) which extends at an angle different from 0° with respect to the longitudinal direction (L), **characterized in that** a sliding sealing device (20), which is capable of being applied to the first movable body (12) in such a way that a relative rotation is made possible between the first body (12) and the second body (14) by a pre-set angle, is arranged on the second end portion (6) of the sealing device.

2. Apparatus (50) according to claim 1, **characterized in that** the sliding seal device (20) has a shaft seal ring.

3. Apparatus (50) according to claim 1, **characterized in that** the first transverse direction (X) is substantially at a right angle to the longitudinal direction (L).

4. Apparatus (50) according to at least one of the preceding claims, **characterized in that** the main body (2) is designed at least in sections in the form of a folding bellows.

5. Apparatus (50) according to at least one of the preceding claims, **characterized in that** a receiving flange (42) is provided on the first end portion (4).

6. Apparatus (50) according to at least one of the preceding claims, **characterized in that** the sealing device (1) is formed in one piece.

7. Apparatus (50) according to at least one of the preceding claims, **characterized in that** the main body (2) and the sliding seal (20) are elements which are separately connected to each other.

8. Apparatus (50) according to at least one of the preceding claims, **characterized in that** a peripheral projection (16), on which at least one portion of the sliding seal (20) is arranged, is arranged on the first movable body (12).

9. Apparatus (50) according to at least one of the preceding claims, **characterized in that** the second end portion (6) is movable with respect to the first end portion (4) in a second transverse direction (Y) which extends at an angle different from 0° with respect to the longitudinal direction (L) and to the first transverse direction (X).

10. Apparatus (50) according to at least one of the preceding claims, **characterized in that** the first end portion (4) is connected in a gas-tight manner to the second body (14) and/or the second end portion (6) is connected in a gas-tight manner to the first movable body (12).

11. An apparatus (50) for the treatment of containers, with a conveying device (52) which conveys the containers to be treated along a pre-set conveying path, with a clean room (54), inside which the containers are conveyed, wherein this clean room (54) is partitioned off from an environment by means of at least one wall (14), with a treatment element (12) which is movable with respect to the wall (14) and which treats the containers in a pre-set manner, wherein a drive (62) for moving this treatment element (12) is arranged at least in sections outside the clean room (54) and the treatment element (12) projects at least in sections into the clean room, and with a sealing device (1) for sealing off an intermediate space formed between the treatment element (12) and the wall (14) of the clean room (54), wherein the sealing device (1) has a main body (2) which is formed from a resilient and gas-tight material and which extends completely in a peripheral direction and through which a portion of the treatment element (12) is guided, and a first end portion (4), which is fastened to the wall (14), and a second end portion (6), which is capable of being applied to the treatment element (12), **characterized in that** the second end portion (6) of the sealing device has arranged on it a sealing element (20) which is capable of being applied to the treatment element (12) in such a way that a relative rotation between the first body (12) and the second body (14) by a pre-set angle of rotation is made possible.

12. An apparatus (1) according to claim 7, **characterized in that** the second end portion (6) is movable with respect to the first end portion in a first transverse direction (X) which is substantially at a right angle to the longitudinal direction (L).

13. An apparatus (1) according to claim 8, **characterized in that** the treatment element is selected from a group of treatment elements which contains closure elements for the closing of containers with closures, filling elements for the filling of containers, sterilization elements for the sterilization of containers, holding elements for the holding of containers and the like.

## Revendications

1. Dispositif (50) pour transformer des ébauches en matière plastique (10) en récipients plastiques (20), avec un dispositif d'étanchéité (1) pour rendre étanches des espaces intermédiaires formés entre des corps (12, 14) mobiles l'un par rapport à l'autre, le dispositif d'étanchéité comportant un corps de base (2), qui est réalisé dans un matériau élastique et étanche aux gaz, est totalement périphérique dans une direction circonférentielle et à travers lequel on peut faire passer une portion du premier des deux corps (12, 14), et une première zone d'extrémité (4), qui peut être fixée au deuxième corps (14), et une deuxième zone d'extrémité (6), qui peut être mise en appui contre le premier corps (12) mobile, la deuxième zone d'extrémité (6) étant mobile par rapport à la première zone d'extrémité (4) dans une première direction transversale (X), qui forme un angle différent de 0° avec la direction longitudinale (L),
**caractérisé en ce**
**qu'**au niveau de la deuxième zone d'extrémité (6) du dispositif d'étanchéité est disposé un dispositif d'étanchéité coulissant (20), qui peut être mis en appui contre le premier corps (12) mobile de manière à permettre une rotation relative entre le premier corps (12) et le deuxième corps (14) selon un angle défini.

2. Dispositif (50) selon la revendication 1, **caractérisé en ce que** le dispositif d'étanchéité coulissant (20) comporte une bague d'étanchéité ondulée.

3. Dispositif (50) selon la revendication 1, **caractérisé en ce que** la première direction transversale (X) est sensiblement perpendiculaire à la direction longitudinale (L).

4. Dispositif (50) selon au moins une des revendications précédentes, **caractérisé en ce que** le corps de base (2) est réalisé au moins par zones sous forme de soufflet.

5. Dispositif (50) selon au moins une quelconque des revendications précédentes, **caractérisé en ce qu'**une collerette de réception (42) est disposée au niveau de la première zone d'extrémité (4).

6. Dispositif (50) selon au moins une des revendications précédentes, **caractérisé en ce que** le dispositif d'étanchéité (1) est réalisé en une seule pièce.

7. Dispositif (50) selon au moins une des revendications précédentes, **caractérisé en ce que** le corps de base (2) et le dispositif d'étanchéité coulissant (20) sont des éléments reliés séparément l'un à l'autre.

8. Dispositif (50) selon au moins une des revendications précédentes, **caractérisé en ce que** sur le premier corps (12) mobile est disposée une saillie (16) périphérique, sur laquelle est disposée au moins une portion du dispositif d'étanchéité coulissant (20).

9. Dispositif (50) selon au moins une des revendications précédentes, **caractérisé en ce que** la deuxième zone d'extrémité (6) est mobile par rapport à la première zone d'extrémité (4) dans une deuxième direction transversale (Y), qui forme un angle différent de 0° avec la direction longitudinale (L) et avec la première direction transversale (X).

10. Dispositif (50) selon au moins une des revendications précédentes, **caractérisé en ce que** la première zone d'extrémité (4) est reliée de manière étanche aux gaz au deuxième corps (14) et/ou la deuxième zone d'extrémité (6) est reliée de manière étanche aux gaz au premier corps (12) mobile.

11. Dispositif (50) pour le traitement de récipients, comportant un dispositif de transport (52) qui transporte les récipients à traiter le long d'une voie de transport définie, comportant une salle blanche (54) à l'intérieur de laquelle les récipients sont transportés, ladite salle blanche (54) étant séparée d'un environnement au moyen d'au moins une paroi (14), comportant un élément de traitement (12), qui est mobile par rapport à la paroi (14) et qui traite les récipients d'une manière définie, un système d'entraînement (62) permettant de déplacer ledit élément de traitement (12) étant disposé au moins par zones en dehors de la salle blanche (54) et l'élément de traitement (12) s'engageant au moins par zones dans la salle blanche, et comportant un dispositif d'étanchéité (1) permettant de rendre étanche un espace intermédiaire se formant entre l'élément de traitement (12) et la paroi (14) de la salle blanche (54), le dispositif d'étanchéité (1) comportant un corps de base (2) étant formé dans un matériau élastique et étanche aux gaz, qui est totalement périphérique dans une direction circonférentielle et à travers lequel passe une portion de l'élément de traitement (12), et une première zone d'extrémité (4), qui est fixée à la paroi (14), et une deuxième zone d'extrémité (6), qui peut être mise en appui contre l'élément de traitement (12), **caractérisé en ce**
**qu'**au niveau de la deuxième zone d'extrémité (6) du dispositif d'étanchéité est disposé un élément d'étanchéité (20), qui peut être mis en appui contre l'élément de traitement (12) de manière à permettre une rotation relative entre le premier corps (12) et le deuxième corps (14) selon un angle de rotation défini.

12. Dispositif (1) selon la revendication 7, **caractérisé en ce que** la deuxième zone d'extrémité (6) est mobile par rapport à la première zone d'extrémité dans une première direction transversale (X) qui est sensiblement perpendiculaire à la direction longitudinale (L).

13. Dispositif (1) selon la revendication 8, **caractérisé en ce que** l'élément de traitement est choisi dans un groupe d'éléments de traitement qui contient des éléments de fermeture pour fermer des récipients avec des fermetures, des éléments de remplissage pour remplir les récipients, des éléments de stérilisation pour stériliser les récipients, des éléments de fixation pour fixer les récipients et tout élément similaire.
